# EUROPEAN PATENT SPECIFICATION

(11) **EP 2 344 489 B1**
(45) Date of publication and mention of the grant of the patent: **22.01.2014**
(21) Application number: 09783000.4
(22) Date of filing: 15.09.2009
(51) Int. Cl.: C07D 461/00

(54) **PROCESS FOR THE PREPARATION OF VINPOCETINE AND APOVINCAMINE**
VERFAHREN ZUR HERSTELLUNG VON VINPOCETIN UND APOVINCAMIN
Procédé pour la préparation de vinpocétine d'apovincamine

(30) Priority: 25.09.2008 EP 08165083
(43) Date of publication of application: 20.07.2011
(73) Proprietor: Linnea S.A., 6595 Riazzino (CH)
(72) Inventor: PARACCHINI, Silvano, CH-6600 Muralto (ti) (CH); LUZZANI, Marcello, I-22030 Lipomo (co) (IT)
(74) Representative: Allaix, Roberto
(86) International application number: PCT/EP2009/061907
(87) International publication number: WO 2010/034647

(56) References cited:
- EP-A- 0 045 918

## Description

### FIELD OF THE INVENTION

The present invention relates to a process for the preparation of vinpocetine and apovincamine and, more particularly, to an improved process for the preparation of vinpocetine and apovincamine.

More in particular, the present invention relates to an improved process for the preparation of vinpocetine and apovincamine through the reaction of the 1-ethyl-1,2,3,4,6,7,12,12b-octahydroindolo[2,3-a]quinolizine-1-carbaldehyde with alkyl haloacetate in an aprotic solvent in the presence of a suitable base.

### BACKGROUND OF THE INVENTION

Vinpocetine is a derivative of the alkaloid vincamine. Vincamine is found in the aerial part of Vinca minor plant and can also be derived from other plant sources such as the Voaconga and the Crioceras Longiflorus. The Vinca minor plant is a creeping root plant which has a long history of use as a traditional tonic to refresh weariness, especially the type associated with advanced age, and also as an astringent, for excessive menses, bleeding gums and mouth sores.

Vinpocetine is the active ingredient of Cavinton and Intelectol. Vinpocetine is held to exhibit an activity on neuronal metabolism by favoring the aerobic glycolysis and promoting the redistribution of the blood flow towards ischemic areas. Vinpocetine is also reported to act to increase cerebral circulation and the use of oxygen.

Vinpocetine is commonly used as an aid to improving memory, as an aid in activities requiring highly focused attention and concentration such as technical writing or computer operation and to combat the symptoms of senile dementia. Vinpocetine has also been reported as showing promising results in the treatment of tinnitus or ringing in the ears as well as other causes of impaired hearing. Vinpocetine is also indicated in the treatment of strokes, menopausal symptoms and macular degeneration. Literature suggests Vinpocetine may also act to improve conditions related to insufficient blood flow to the brain including vertigo and Ménière's disease, difficulty in sleeping, mood changes and depression.

Vinpocetine is represented by the following formula (I). The chemical name of vinpocetine is (3α,16α)-eburnamenine-14-carboxylic acid ethyl ester. Apovincamine is the corresponding methyl ester of the (3α,16α)-eburnamenine-14-carboxylic acid.

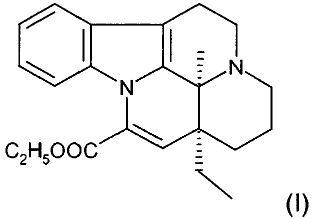

The US patent No. 4,379,935 describes a method for the preparation of vincamine and related indole alkaloids, including vinpocetine and apovincamine, starting from the compound of formula (II), through the formation of the intermediate glycidic ester (III). The preparation method of the compound of formula (II) has been described by W. Oppolzer et al., Helv. Chim. Acta, 60(5), 1801 (1977). The chemical name of the compound of formula (II) is (1R,5S,12bS)-1-ethyl-1,2,3,4,6,7,12,12b-octahydroindolo[2,3-a]quinolizine-1-carbaldehyde. Hereinbelow, compound of formula (II) will be named Oppolzer's aldehyde.

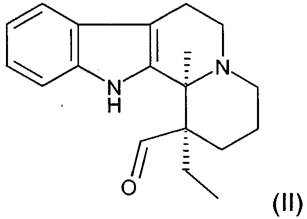

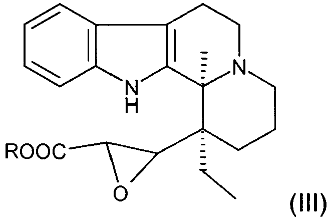

The preparation process described in US patent No. 4,379,935 requires a first step, wherein the Oppolzer's aldehyde (II) is converted to the glycidic ester (III) by treatment with a haloester in the presence of a base in anhydrous ethers or hydrocarbons, and a second step wherein the glycidic ester (III) is reacted with a Lewis acid in toluene or with a diluted mineral acid, to get a mixture of vincamine and related alkaloids, such as, ethyl vincaminate, apovincamine, ethyl apovincaminate (vinpocetine), ethyl epivincaminate, and vincanol.

### SUMMARY OF THE INVENTION

The Applicant has faced the problem of improving the process for the preparation of vinpocetine and apovincamine starting from Oppolzer's aldehyde.

The Applicant has found that the process described in the art has several drawbacks.

First, the prior art process requires a two-step reaction, which makes the whole process more complex and expensive than a single step reaction process.

Further, the yield of vinpocetine and apovincamine is particularly low, with a high amount of by-products which need to be separated and, if possible, recycled.

Moreover, the prior art process results in a mixture of several end-products and by-products, which requires a further separation step, which increases the time and cost of the whole process.

All the above disadvantages made the whole prior art process economically not convenient and not practicable.

The Applicant has found that the above mentioned problems may be overcome by reacting the Oppolzer's aldehyde with alkyl haloacetate in a polar aprotic solvent, preferably selected from the group consisting of tetrahydrofuran (THF), 1,4-dioxane, dimethyl formamide (DMF), dimethyl acetamide (DMA), dimethyl sulfoxide (DMSO), acetonitrile (AN), and N-methylpyrrolidone (NMP) in the presence of a suitable base.

The Applicant has surprisingly found that the process of the present invention allows to obtain vinpocetine and apovincamine with higher yields that the prior art process.

Moreover, the Applicant has surprisingly found that the process of the present invention allows to directly obtain vinpocetine and apovincamine with a single step reaction without the formation and/or isolation of any intermediate compound and without the need of a further reaction with Lewis acids or mineral acids.

Further, the Applicant has surprisingly found that the process of the present invention allows to directly obtain vinpocetine and apovincamine with a low amount of by-products and without the presence of other end-products like ethylvincaminate and vincamine.

According to a preferred embodiment of the present invention, a non polar solvent is added to the reaction mixture before the completion of the reaction.

The Applicant has surprisingly found that the addition of a non polar solvent in the process of the present invention allows to further increase the yield of the end products vinpocetine and apovincamine.

Accordingly, the present invention relates to a process for the preparation of vinpocetine and apovincamine, wherein said process comprises the steps of:
(1) preparing a solution of the compound of formula (II) below and an organic or inorganic base in a polar aprotic solvent, and 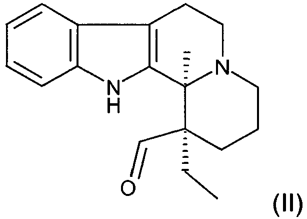
(2) adding an alkyl haloacetate to the solution of step (1) under controlled temperature conditions.

### DETAILED DESCRIPTION OF THE INVENTION

The organic or inorganic base can be any basic compound able to activate the Darzens' reaction between the Oppolzer's aldehyde and the alkyl haloacetate.

Preferably, the organic or inorganic base can be sodium hydride, potassium hydride, sodium hydroxide, potassium hydroxide, sodium amide, potassium amide, sodium or potassium alkoxides, such as, for example, sodium ethoxide, potassium ethoxide, potassium iso-propoxide, potassium t-butoxide; lithium alkoxides, such as, for example, lithium ethoxide, lithium ethoxide, lithium iso-propoxide, lithium t-butoxide; lithium dialkylamides, such as, for example, lithium dimethylamide, lithium diethylamide, lithium diisopropylamide, lithium dicyclohexylamide; and lithium alkyls, such as, for example methyl lithium, ethyl lithium, propyl lithium, and butyl lithium.

According to a preferred embodiment the organic or inorganic base is sodium ethoxide, potassium ethoxide, potassium t-butoxide, sodium hydride and potassium hydride.

Advantageously, the polar aprotic solvent can be tetrahydrofuran (THF), 1,4-dioxane, dimethyl formamide (DMF), dimethyl acetamide (DMA), dimethyl sulfoxide (DMSO), acetonitrile (AN), and N-methylpyrrolidone (NMP).

According to a preferred embodiment the polar aprotic solvent is tetrahydrofuran, dimethyl formamide, and N-methylpyrrolidone.

The solution of step (1) can preferably comprise from 1 to 20, more preferably from 5 to 15 parts by weight of Oppolzer's aldehyde per 100 parts by volume of polar aprotic solvent.

Further, the solution of step (1) can preferably comprise from 1 to 20, more preferably from 5 to 15 parts by weight of organic or inorganic base per 100 parts by volume of polar aprotic solvent.

Preferably, the organic or inorganic base employed in step (1) is added in molar excess with respect to the molar amount of Oppolzer's aldehyde. The molar amount of organic or inorganic base preferably ranges from 1.5 to 10, more preferably from 2 to 8 moles per mole of Oppolzer's aldehyde.

The order of addition of the components, namely the Oppolzer's aldehyde and the organic or inorganic base, to the polar aprotic solvent to prepare the solution of step (1) is not particularly relevant. The components can be added together or separately to the proper volume of solvent, or two solutions can be separately prepared and then mixed together. However, this latter method is preferable, in particular, by adding a previously prepared solution of Oppolzer's aldehyde to a previously prepared solution of organic or inorganic base.

The preparation of the solution of step (1) of the process of the present invention is preferably made maintaining the temperature of from -20° to +20°C, more preferably from -15 to +15°C. Advantageously, the solution is maintained at a temperature is ranging from -10° to +10°C.

The addition of alkyl haloacetate according to step (2) of the process of the present invention is preferably made maintaining the temperature in the range of from -20° to +10°C, more preferably from -15 to +5°C. Advantageously, the reaction temperature is ranging from -10° to 0°C.

The alkyl haloacetate employed in step (2) of the present invention can be an alkyl bromoacetate, alkyl chloroacetate, or alkyl iodoacetate. The alkyl group can be a methyl or an ethyl group. Methyl chloroacetate and ethyl chloroacetate are preferably employed.

The alkyl haloacetate employed in step (2) is preferably added in molar excess with respect to the molar amount of Oppolzer's aldehyde. The molar amount of alkyl haloacetate preferably ranges from 1.1 to 10, more preferably from 1.5 to 8 moles per mole of Oppolzer's aldehyde.

The Applicant has surprisingly found that according to the process of the present invention, the alkyl haloacetate reacted with the Oppolzer's aldehyde giving the desired compound, namely vinpocetine or apovincamine, without the need of further reaction steps.

Advantageously, a non polar solvent is added to the reaction mixture during step (2) before the completion of the reaction. Preferably, the non polar solvent is a carboxylic acid alkyl ester, more preferably a carboxylic acid alkyl ester having from 2 to 8 carbon atoms, such as, for example, methyl formate, ethyl formate, methyl orthoformate, ethyl orthoformate, propyl formate, methyl acetate, ethyl acetate, propyl acetate, ethyl propionate, butyl butyrate, and the like.

The Applicant has surprisingly found that the use of such a non polar solvent in the process of the present invention allows to further increase the yield of the end products vinpocetine and apovincamine together with a reduction of by-products.

The non polar solvent optionally employed in step (2) is preferably added in an amount of from 5 to 50 % v/v, more preferably from 10 to 30 % v/v with respect to the initial volume of the polar aprotic solvent.

The end product can be isolated with conventional procedures known to a man skilled in the art, which include distillation, precipitation, filtration, and exsiccation.

From the detailed description above, it is therefore readily apparent that the process of the present invention shows several advantages with respect to those already described in the art.

For better illustrating the invention the following non-limiting examples are now given.

### Comparison Example A

The comparison example A was made following the procedure of Example 1 of US 4,379,935.
A.1 Oppolzer's aldehyde (2.0 g, 0.007 mol) and ethyl chloroacetate (1.1 ml, 1.3 g, 0.01 mol) were dissolved in anhydrous ethyl ether (100 ml) in a tank of about 250 ml provided with stirrer, dropper, condenser, thermometer and heater. The reaction mixture has been cooled at a temperature between 0° and 5°C with an ice water bath. Sodium amide (0.52 g) was added in four equal portions along 30 minutes. The reaction mixture was maintained for one hour at about 0°C and one hour at about 25°C. No reaction was observed and the Oppolzer's aldehyde remained unreacted in the reaction mixture.
A.2 The procedure of example A.1 was repeated, but using anhydrous toluene instead of ethyl ether. No reaction was observed and the Oppolzer's aldehyde remained unreacted in the reaction mixture.
A.3 The procedure of example A.1 was repeated, but using sodium ethoxide (0.91 g) instead of sodium amide. The partial conversion (80% m/m) of the Oppolzer's aldehyde was observed. The reaction product was extracted with CH2Cl2 following the procedure described in example 1.1 of US 4,379,935, so obtaining about 1.1 g of raw product.
   The raw product was dissolved in anhydrous toluene (30 ml). The resulting solution was added with BF3 etherate (10 ml) and heated to 80°C under stirring for two hours. At the end of the reaction the solution was cooled, filtered and concentrated under vacuum. The residue was treated by column chromatography as described in Example 1.2-a of US 4,379,935.
   From the column eluate, vinpocetine was obtained with a very low ponderal yield (3 % w/w) based on the starting aldehyde.
A.4 The procedure of example A.3 was repeated, but using anhydrous toluene instead of ethyl ether. The same partial conversion (80% m/m) of the Oppolzer's aldehyde and the same vinpocetine yield (3% w/w) was observed.

### Example 1

Oppolzer's aldehyde (10.0 g, 0.035 mol) was dissolved in THF (60 ml). Separately, sodium ethoxide (6.0 g, 0.088 mol) was dissolved in THF (40 ml). The two solutions were joined maintaining the temperature within the range of from 0° to +5°C. Then, ethyl chloroacetate (5.6 ml, 6.44 g, 0.052 mol) was added dropwise over 30 min, under stirring and controlling the temperature below 10°C.

After completion of the reaction, the reaction mixture was distilled under reduced pressure. The residual was added with water and dichloromethane. The organic phase was separated and subjected to chromatography on a silicon dioxide column using as eluent a mixture of 98% dichloromethane and 2% methanol.

Pure vinpocetine was obtained (4.68 g) with a yield of 46.8% w/w and 38.2% m/m based on the starting aldehyde.

### Example 2

The procedure of example 1 was repeated, but using DMF instead of THF and sodium hydride (5.7 g, 0.143 mol) instead of sodium ethoxide.

Pure vinpocetine was obtained (6.50 g) with a yield of 65.0% w/w and 52.5% m/m based on the starting aldehyde.

### Example 3

The procedure of example 1 was repeated, but using NMP instead of THF and sodium hydride (5.7 g, 0.143 mol) instead of sodium ethoxide.

Pure vinpocetine was obtained (5.40 g) with a yield of 54.0% w/w and 43.5% m/m based on the starting aldehyde.

### Example 4

The procedure of example 2 was repeated, but using ethyl bromoacetate instead of ethyl chloroacetate (same molar amount).

Pure vinpocetine was obtained (5.30 g) with a yield of 53.0% w/w and 42.5% m/m based on the starting aldehyde.

### Example 5

The procedure of example 2 was repeated, but using ethyl iodoacetate instead of ethyl chloroacetate (same molar amount).

Pure vinpocetine was obtained (4.90 g) with a yield of 49.0% w/w and 37.5% m/m based on the starting aldehyde.

### Example 6

Oppolzer's aldehyde (10.0 g, 0.035 mol) was dissolved in THF (60 ml) - 5°C. Separately, sodium ethoxide (6.0 g, 0.088 mol) was dissolved in THF (40 ml). The two solutions were joined maintaining the temperature within the range of from -5° to -10°C. Then, ethyl chloroacetate (5.6 ml, 6.44 g, 0.052 mol) was added dropwise over 30 min, under stirring and controlling the temperature below -5°C.

Before completion of the reaction, ethyl acetate (30 ml) is added and the temperature of the reaction mixture is raised to 50°C for two hours. Then, the reaction mixture was distilled under reduced pressure.

The residual was added with water and dichloromethane. The organic phase was separated and subjected to chromatography on a silicon dioxide column using as eluent a mixture of 98% dichloromethane and 2% methanol.

Pure vinpocetine was obtained (7.94 g) with a yield of 79.4% w/w and 64.7% m/m based on the starting aldehyde.

### Example 7

Oppolzer's aldehyde (10 g, 0.035 mol) was dissolved in THF (60 ml) -5°C. Separately, sodium methoxide (4.9 g, 0.090 mol) was dissolved in THF (40 ml). The two solutions were joined maintaining the temperature within the range of from -5° to -10°C. Then, methyl chloroacetate (4.7 ml, 5.77 g, 0.052 mol) was added dropwise over 30 min, under stirring and controlling the temperature below -5°C.

Before completion of the reaction, methyl acetate (50 ml) is added and the temperature of the reaction mixture is raised to 50°C for two hours. Then, the reaction mixture was distilled under reduced pressure.

The residual was added with water and dichloromethane. The organic phase was separated and subjected to chromatography on a silicon dioxide column using as eluent a mixture of 98% dichloromethane and 2% methanol.

Pure apovincamine was obtained (8.35 g) with a yield of 83.5% w/w and 70.9% m/m based on the starting aldehyde.

## Claims

1. A process for the preparation of vinpocetine and apovincamine, wherein said process comprises the steps of:
(1) preparing a solution of the compound of formula (II) below and an organic or inorganic base in a polar aprotic solvent, and 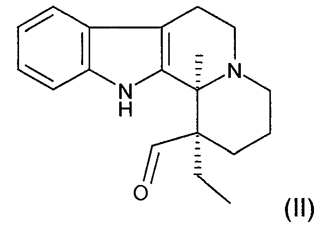
(2) adding an alkyl haloacetate to the solution of step (1) under controlled temperature conditions.

2. The process according to claim 1, wherein said organic or inorganic base is selected from the group consisting of sodium hydride, potassium hydride, sodium hydroxide, potassium hydroxide, sodium amide, potassium amide, sodium or potassium alkoxides, lithium alkoxides, lithium dialkylamides, and lithium alkyls.

3. The process according to claim 2, wherein said organic or inorganic base is selected from the group consisting of sodium ethoxide, potassium ethoxide, potassium t-butoxide, sodium hydride and potassium hydride.

4. The process according to any one of preceding claims, wherein said polar aprotic solvent is selected from the group consisting of tetrahydrofuran (THF), 1,4-dioxane, dimethyl formamide (DMF), dimethyl acetamide (DMA), dimethyl sulfoxide (DMSO), acetonitrile (AN), and N-methylpyrrolidone (NMP).

5. The process according to claim 4, wherein said polar aprotic solvent is selected from the group consisting of tetrahydrofuran, dimethyl formamide, and N-methylpyrrolidone.

6. The process according to any one of preceding claims, wherein said solution of step (1) comprises from 1 to 20 parts by weight of said compound of formula (II) per 100 parts by volume of said polar aprotic solvent.

7. The process according to any one of preceding claims, wherein said solution of step (1) comprises from 1 to 20 parts by weight of said organic or inorganic base per 100 parts by volume of said polar aprotic solvent.

8. The process according to any one of preceding claims, wherein said solution of step (1) is prepared maintaining the solution temperature of from-20° to +20°C.

9. The process according to any one of preceding claims, wherein the addition of alkyl haloacetate in step (2) is made maintaining the solution temperature in the range of from -20° to +10°C.

10. The process according to claim 9, wherein the addition of alkyl haloacetate in step (2) is made maintaining the solution temperature in the range of from -10° to 0°C.

11. The process according to any one of preceding claims, wherein said alkyl haloacetate is selected from the group consisting of alkyl bromoacetate, alkyl chloroacetate, and alkyl iodoacetate.

12. The process according to claim 11, wherein said alkyl haloacetate is selected from the group consisting of methyl chloroacetate and ethyl chloroacetate.

13. The process according to any one of preceding claims, wherein said alkyl haloacetate is added in molar excess with respect to the molar amount of Oppolzer's aldehyde.

14. The process according to any one of preceding claims, wherein a non polar solvent is added to the reaction mixture during step (2) before the completion of the reaction.

15. The process according to claim 14, wherein said non polar solvent is selected from the group consisting of methyl formate, ethyl formate, methyl orthoformate, ethyl orthoformate, propyl formate, methyl acetate, ethyl acetate, propyl acetate, ethyl propionate, and butyl butyrate.

16. The process according to any one of claims 14 and 15, wherein said non polar solvent is added in an amount of from 5 to 50 % v/v with respect to the initial volume of the polar aprotic solvent.

## Patentansprüche

1. Verfahren zur Herstellung von Vinpocetin und Apovincamin, wobei das Verfahren die folgenden Schritte umfasst:
(1) Herstellung einer Lösung der Verbindung der nachstehenden Formel (II) und einer organischen oder anorganischen Base in einem polaren aprotischen Lösungsmittel, und 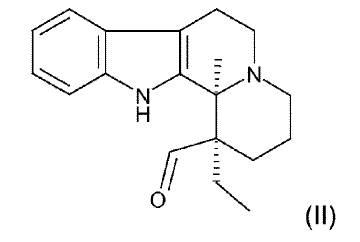
(2) Zugabe eines Alkylhalogenacetats zu der Lösung aus Schritt (1) unter kontrollierten Temperaturbedingungen.

2. Verfahren nach Anspruch 1, wobei die organische oder anorganische Base ausgewählt ist aus der Gruppe bestehend aus Natriumhydrid, Kaliumhydrid, Natriumhydroxid, Kaliumhydroxid, Natriumamid, Kaliumamid, Natrium- oder Kaliumalkoxiden, Lithiumalkoxiden, Lithiumdialkylamiden und Lithiumalkylen.

3. Verfahren nach Anspruch 2, wobei die organische oder anorganische Base ausgewählt ist aus der Gruppe bestehend aus Natriumethanolat, Kaliumethanolat, Kalium-tert-butanolat, Natriumhydrid und Kaliumhydrid.

4. Verfahren nach einem der vorhergehenden Ansprüche, wobei das polare aprotische Lösungsmittel ausgewählt ist aus der Gruppe bestehend aus Tetrahydrofuran (THF), 1,4-Dioxan, Dimethylformamid (DMF), Dimethylacetamid (DMA), Dimethlysulfoxid (DMSO), Acetonitril (AN) und N-Methylpyrrolidon (NMP).

5. Verfahren nach Anspruch 4, wobei das polare aprotische Lösungsmittel ausgewählt ist aus der Gruppe bestehend aus Tetrahydrofuran, Dimethylformamid und N-Methylpyrrolidon.

6. Verfahren nach einem der vorhergehenden Ansprüche, wobei die Lösung aus Schritt (1) 1 bis 20 Gewichtsteile der Verbindung der Formel (II) pro 100 Volumenteile des protischen Lösungsmittels umfasst.

7. Verfahren nach einem der vorhergehenden Ansprüche, wobei die Lösung aus Schritt (1) 1 bis 20 Gewichtsteile der organischen oder anorganischen Base pro 100 Volumenteile des protischen Lösungsmittels umfasst.

8. Verfahren nach einem der vorhergehenden Ansprüche, wobei die Lösung aus Schritt (1) hergestellt wird, während man die Lösungstemperatur im Bereich von -20 bis +20 °C hält.

9. Verfahren nach einem der vorhergehenden Ansprüche, wobei die Zugabe des Alkylhalogenacetats in Schritt (2) erfolgt, während man die Lösungstemperatur im Bereich von -20 bis +10 °C hält.

10. Verfahren nach Anspruch 10, wobei die Zugabe des Alkylhalogenacetats in Schritt (2) erfolgt, während man die Lösungstemperatur im Bereich von -10 bis 0 °C hält.

11. Verfahren nach einem der vorhergehenden Ansprüche, wobei das Alkylhalogenacetat ausgewählt ist aus der Gruppe bestehend aus Alkylbromacetat, Alkylchloracetat und Alkyliodacetat.

12. Verfahren nach Anspruch 11, wobei das Alkylhalogenacetat ausgewählt ist aus der Gruppe bestehend aus Methylchloracetat und Ethylchloracetat.

13. Verfahren nach einem der vorhergehenden Ansprüche, wobei das Alkylhalogenacetat im molaren Überschuss bezüglich der molaren Menge an Oppolzers Aldehyd zugegeben wird.

14. Verfahren nach einem der vorhergehenden Ansprüche, wobei man während Schritt (2) dem Reaktionsgemisch vor Beendigung der Umsetzung ein unpolares Lösungsmittel zufügt.

15. Verfahren nach Anspruch 14, wobei das unpolare Lösungsmittel ausgewählt ist aus der Gruppe bestehend aus Methylformiat, Ethylformiat, Methylorthoformiat, Ethylorthoformiat, Propylformiat, Methylacetat, Ethylacetat, Propylacetat, Ethylpropionat und Butylbutyrat.

16. Verfahren nach einem der Ansprüche 14 und 15, wobei das unpolare Lösungsmittel in einer Menge von 5 bis 50 Vol.-% bezüglich des Anfangsvolumens des polaren aprotischen Lösungsmittels zugegeben wird.

## Revendications

1. Procédé pour la préparation de vinpocétine et d'apovincamine, lequel procédé comprend les étapes consistant à:
(1) préparer une solution du composé de formule (II) ci-dessous et d'une base organique ou inorganique dans un solvant aprotique polaire, et 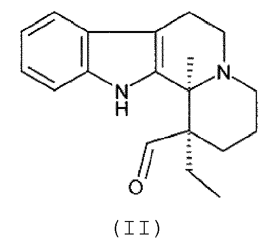
(2) ajouter un halogénoacétate d'alkyle à la solution de l'étape (1) dans des conditions régulées en température.

2. Procédé selon la revendication 1, dans lequel ladite base organique ou inorganique est choisie dans le groupe constitué par l'hydrure de sodium, l'hydrure de potassium, l'hydroxyde de sodium, l'hydroxyde de potassium, le sodium-amide, le potassium-amide, les alkylates de sodium ou de potassium, les alkylates de lithium, les lithium-dialkylamides, et les alkyl-lithiums.

3. Procédé selon la revendication 2, dans lequel ladite base organique ou inorganique est choisie dans le groupe constitué par l'éthylate de sodium, l'éthylate de potassium, le t-butylate de potassium, l'hydrure de sodium et l'hydrure de potassium.

4. Procédé selon l'une quelconque des revendications précédentes, dans lequel ledit solvant aprotique polaire est choisi dans le groupe constitué par le tétrahydrofurane (THF), le 1,4-dioxane, le diméthylformamide (DMF), le diméthylacétamide (DMA), le diméthylsulfoxyde (DMSO), l'acétonitrile (AN), et la N-méthylpyrrolidone (NMP).

5. Procédé selon la revendication 4, dans lequel ledit solvant aprotique polaire est choisi dans le groupe constitué par le tétrahydrofurane, le diméthylformamide, et la N-méthylpyrrolidone.

6. Procédé selon l'une quelconque des revendications précédentes, dans lequel ladite solution de l'étape (1) comprend de 1 à 20 parties en poids dudit composé de formule (II) pour 100 parties en volume dudit solvant aprotique polaire.

7. Procédé selon l'une quelconque des revendications précédentes, dans lequel ladite solution de l'étape (1) comprend de 1 à 20 parties en poids de ladite base organique ou inorganique pour 100 parties en volume dudit solvant aprotique polaire.

8. Procédé selon l'une quelconque des revendications précédentes, dans lequel ladite solution de l'étape (1) est préparée avec maintien de la température de la solution dans la plage allant de -20°C à +20°C.

9. Procédé selon l'une quelconque des revendications précédentes, dans lequel l'addition d'halogénoacétate d'alkyle dans l'étape (2) est effectuée avec maintien de la température de la solution dans la plage allant de -20°C à +10°C.

10. Procédé selon la revendication 9, dans lequel l'addition d'halogénoacétate d'alkyle dans l'étape (2) est effectuée avec maintien de la température de la solution dans la plage allant de -10°C à 0°C.

11. Procédé selon l'une quelconque des revendications précédentes, dans lequel ledit halogénoacétate d'alkyle est choisi dans le groupe constitué par un bromoacétate d'alkyle, un chloroacétate d'alkyle, et un iodoacétate d'alkyle.

12. Procédé selon la revendication 11, dans lequel ledit halogénoacétate d'alkyle est choisi dans le groupe constitué par le chloroacétate de méthyle et le chloroacétate d'éthyle.

13. Procédé selon l'une quelconque des revendications précédentes, dans lequel ledit halogénoacétate d'alkyle est ajouté en excès molaire par rapport à la quantité molaire d'aldéhyde d'Oppolzer.

14. Procédé selon l'une quelconque des revendications précédentes, dans lequel un solvant non polaire est ajouté au mélange réactionnel au cours de l'étape (2) avant que la réaction soit terminée.

15. Procédé selon la revendication 14, dans lequel ledit solvant non polaire est choisi dans le groupe constitué par le formiate de méthyle, le formiate d'éthyle, l'orthoformiate de méthyle, l'orthoformiate d'éthyle, le formiate de propyle, l'acétate de méthyle, l'acétate d'éthyle, l'acétate de propyle, le propionate d'éthyle, et le butyrate de butyle.

16. Procédé selon l'une quelconque des revendications 14 et 15, dans lequel ledit solvant non polaire est ajouté en une quantité de 5 à 50 % v/v par rapport au volume initial du solvant aprotique polaire.
